# EUROPEAN PATENT SPECIFICATION

(11) **EP 4 324 836 B1**
(45) Date of publication and mention of the grant of the patent: **22.07.2026**
(21) Application number: 22749272.5
(22) Date of filing: 17.02.2022
(51) Int. Cl.: C07F 5/02, A61K 31/69, A61P 35/00, A61P 35/04

(54) **NOVEL BORON CARRIER, PREPARATION METHOD AND PHARMACEUTICAL FORMULATION THEREOF**
NEUARTIGER BORTRÄGER, HERSTELLUNGSVERFAHREN UND PHARMAZEUTISCHE FORMULIERUNG DARAUS
NOUVEAU SUPPORT DE BORE, PROCÉDÉ DE PRÉPARATION ET FORMULATION PHARMACEUTIQUE DE CELUI-CI

(30) Priority: 08.02.2021 CN 202110173023
(43) Date of publication of application: 21.02.2024
(73) Proprietor: Peking University, Beijing 100871 (CN)
(72) Inventor: XIANG, Jing, Beijing 1000871 (CN)
(74) Representative: Huang, Liwei
(86) International application number: PCT/CN2022/076651
(87) International publication number: WO 2022/166993

(56) References cited:
- WO-A2-2016/205790
- CN-A- 104 203 961
- CN-A- 109 053 781
- CN-A- 112 979 686
- SHERVINGTON LEROY ET AL: "A novel series of phenolic temozolomide (TMZ) esters with 4 to 5-fold increased potency, compared to TMZ, against glioma cells irrespective of MGMT expression", vol. 10, no. 30, 6 May 2020 (2020-05-06), pages 17561 - 17570, XP055905966, Retrieved from the Internet <URL:https://pubs.rsc.org/en/content/articlepdf/2020/ra/d0ra02686g> DOI: 10.1039/D0RA02686G
- SHERVINGTON LEROY, INGHAM OLIVER, SHERVINGTON AMAL: "A novel series of phenolic temozolomide (TMZ) esters with 4 to 5-fold increased potency, compared to TMZ, against glioma cells irrespective of MGMT expression", RSC ADVANCES, vol. 10, no. 30, 6 May 2020 (2020-05-06), pages 17561 - 17570, XP055905966, DOI: 10.1039/D0RA02686G
- JIYUAN LI;ZHIYU TU;ZHIBO LIU: "Development History of Boron Delivery Agents", SCIENTIA SINICA(CHIMICA), vol. 50, no. 10, 30 September 2020 (2020-09-30), pages 1296 - 1319, XP055957135, ISSN: 1674-7224, DOI: 10.1360/SSC-2020-0114

## Description

### Technical Field

The present invention belongs to the field of medical technology, and relates to a novel boron carrying agent, a preparation method and a pharmaceutical formulation thereof, in particular, to 3,4-dihydro-3-methyl-4-oxoimidazo[5,1-D]-1,2,3,5-tetrazine-8-carboxylic acid [4-(4,4,5,5-tetramethyl-1,3,2-diaza-borolan-2-yl)phenyl ester, a preparation method and a pharmaceutical formulation thereof.

### Background Art

Tumors, especially malignant tumors, are diseases that seriously endanger human health in the world today. Its mortality rate is second only to cardiovascular disease, ranking second among the mortality rates of various diseases. In recent years, its morbidity has shown a significant upward trend. According to current cancer incidence trends, the number of new cancer patients worldwide will reach 15 million per year.

Glioma is a tumor that occurs in the neuroectoderm, and thus it is also named as neuroectodermal tumor or neuroepithelial tumor. Tumors originate from neurostromal cells, namely glia, ependyma, choroid plexus epithelium, and neural parenchymal cells, i.e., neurons. Most tumors originate from different types of glia. However, based on their histogenesis and similar biological characteristics, various tumors arising in the neuroectoderm are generally called gliomas.

There are many classification methods for glioma, and clinical workers often use the relatively simple Kernohan classification method. Among various types of gliomas, astrocytoma accounts for the most, followed by glioblastoma, and successively medulloblastoma, ependymoma, oligodendroglioma, pineal tumor, mixed glioma, choroid plexus papillomas, unclassified gliomas, and neuronal tumors. The predilection sites of various types of gliomas are different. For example, astrocytomas are more common in the cerebral hemispheres in adults and in the cerebellum in children; glioblastomas almost always occur in the cerebral hemispheres; medulloblastomas occur in the cerebellar vermis; and ependymomas are more common in the 4th ventricle.

At present, the treatment methods for glioma are classified into surgery, radiotherapy and chemotherapy according to the effect. Since gliomas grow invasively and have unclear boundaries with surrounding tissues, even if a microscope is used during surgery, a small amount of tumor cells will inevitably be left over, leading to tumor recurrence. Although new progress has been made in chemotherapy, it is difficult for systemic chemotherapy to pass through the blood-brain barrier and achieve an effective drug concentration in the local area of the tumor. Moreover, high-dose chemotherapy causes obvious systemic toxic and side effects, such as bone marrow suppression, reduction of white blood cell and platelet, pulmonary fibrosis, etc. Although each treatment modality has made significant progress, and the combined therapy of the three methods can prolong the patient's survival, glioma is still difficult to be cured. In recent years, new therapies such as boron neutron capture therapy have been adapted from the laboratory to the clinic.

Boron neutron capture therapy (BNCT) is a new method for treating tumors. It was originally proposed by Locher from the United States in 1936. A boron (¹⁰B) carrier having specific affinity for tumors is injected into human body, and accumulated in tumor cells, followed by irradiation with super-heated low-energy neutrons (1eV~10keV), ¹⁰B nuclei reacts with neutrons, and the α particles and ⁷Li³⁺ produced will kill tumor cells: ¹⁰B+¹n→[¹¹B]→⁴He²⁺ (α)+⁷Li³⁺+2.31 MeV. Studies have shown that the neutron reaction cross-section of ¹⁰B in tumor cells is 3800 b (barn), and neutron capture reactions can also occur with hydrogen (¹H) and nitrogen (¹⁴N) in normal cells around the tumor. But the neutron capture reaction cross-sections of ¹H and ¹⁴N are only 0.332 b and 1.82 b, respectively. Thus, compared to the tissues around the tumor, the ¹⁰B reaction cross-section to neutrons in the tumor cells is large and the toxic and side effects are relatively low. However, it was not until the 1980s that major breakthroughs were made in the preparation of boron compound carriers and the problem of thermal neutron sources was simultaneously solved, allowing BNCT to enter the clinical practical stage. Clinically, BNCT is mainly used for treating cancers such as cerebral tumors, gliomas, and melanomas.

The boron carrying agents used in BNCT are a series of boron-containing compounds being passive or active in targeting whose molecules can be selectively taken up by tumor cells. The ideal boron carrying agent should have the following features: 1) The boron dose must meet the requirements, at least ensuring that each gram of tumor tissue absorbs about 10-30 µg¹⁰B, that is, each tumor cell contains 10^{9 10}B atoms, and during the irradiation period, the radiation dose of boron within the tumor cells should be kept above 1 Gy. 2) The ratio of the boron concentration of tumor cells to normal tissue and the ratio of boron concentration of tumor cells to blood must be guaranteed to be equal to or above 3 to ensure the targeting performance of the boron carrying agent. 3) In addition to being taken up by tumor cells at the dividing stage, it can also be taken up by tumor cells. 4) It is non-toxic and has favorable water solubility.

The second generation BNCT reagents are a series of boron-containing organic small molecule compounds being passive in targeting, whose molecules can be selectively absorbed by tumor cells. In terms of clinical treatment, there are two boron-containing medicines approved by the US FDA:
1) Sodium mercaptododecaborate (BSH: Na₂B₁₂H₁₁SH): BSH is a water-soluble compound containing 12 B atoms in the molecule. Experience has proven that BSH has the best aggregation effect in the treatment of cerebral tumors. It was originally synthesized by Miller et al. in 1964 and was first used for the treatment of cerebral tumors in 1968. BNCT irradiation was adopted during craniotomy and achieved favorable therapeutic effect. In 2018, Goeun Choi et al. combined layered double hydroxide (LDH) with BSH. After U87 cells absorbed BSH-LDH nanoparticles, the ¹⁰B content was 42.4 µg/10⁶ cells, which was approximately 2000 times larger than the minimum boron requirement of BNCT. The T/B value of ¹⁰B in mice of the BSH-LDH group was also 4.4 times higher than that of the BSH group within 2 hours. Excellent neutron capture efficiency was also finally produced under the condition of 30 µg ¹⁰B mL⁻¹.
2) ¹⁰B-p-Dicarboxylboronic acid phenylalanine (BPA: ¹⁰BC₉H₁₂NO₄). BPA is a liposoluble compound with a structure similar to phenylalanine. Since tumor cells have strong metabolism and require more phenylalanine than normal cells, tumor cells can actively take up large amounts of BPA. However, because its structure contains boron, BPA is not involved in protein synthesis. Based on the hypothesis that BPA is preferentially taken up by melanin-synthesizing cells, it was initially administered intravenously to treat patients with cutaneous melanoma. Experiments by Coderre et al. in 1990 showed that BPA was also taken up by other types of tumor cells, including rat cerebral 9L gliosarcoma. Mallesh et al. found that the fructose complex of BPA (BPA-F) can significantly improve its water solubility and increase its solubility in the blood. It can be injected intravenously. Therefore, BPA quickly entered the clinical stage and is used for treating patients with malignant melanoma and cerebral glioma.

Due to defects at the level of molecular property, BPA and BSH are not specific enough for tumor cells and cannot meet the aforementioned ideal standards for boron carrying agent. The major challenges in the development of boron carrying agents are the need to selectively target tumor cells and deliver therapeutic concentrations of boron with minimal normal tissue uptake and retention. Directions to improve the selectivity of boron carrying agents include integration with tumor-targeting groups, such as unnatural amino acids, polyamines, polypeptides, proteins, antibodies, nucleosides, sugars, porphyrins, liposomes, and nanoparticles.

One of the main indications for boron neutron capture therapy is cerebral glioma and head and neck tumor. Medicines need to penetrate the blood-brain barrier to reach cerebral gliomas. Thus, subsequent research on boron carrying agents needs to transport sufficient doses of ¹⁰B molecules into tumor cells and maintain a relatively high T/N value. Among them, effectively killing glioma cells without destroying normal brain tissue is more challenging than the treatment of malignant tumors in other locations. The blood-brain barrier blocks drugs with molecular weight greater than 200 Da, and the highly infiltrative nature of glioma cells and their genomic heterogeneity make it more difficult to treat cerebral gliomas. WO 2016/205790 A2 discloses boron containing compounds used for the treatment of cancer.

In order to solve the problem of BPA and BSH's insufficient specificity for tumor cell, it is urgent to develop a novel boron carrying agent medicine molecule.

### Summary

One object of the present invention is to provide a novel boron carrying agent and a pharmaceutically acceptable salt thereof.

The technical solutions of the present invention for achieving the above object are:

In one aspect, the boron carrying agent or a pharmaceutically acceptable salt thereof according to the present invention is 3,4-dihydro-3-methyl-4-oxoimidazo[5,1-D]-1,2,3,5-tetrazine-8-carboxylic acid [4-(4,4,5,5-tetramethyl-1,3,2-diaza-borolan-2-yl)phenyl ester and a pharmaceutically acceptable salt thereof, and the specific molecular formula is:

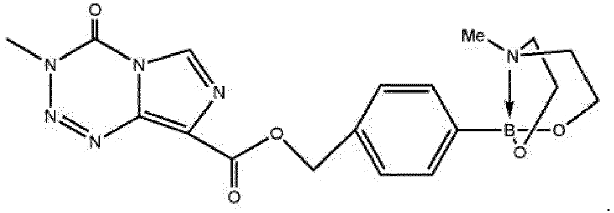

In another aspect, another object of the present invention is to provide a pharmaceutical preparation, which comprises: (1) the boron carrying agent or the pharmaceutically acceptable salt thereof according to the present invention; and (2) one or more pharmaceutically acceptable carriers or excipients.

In a third aspect, the present invention also provides a preparation method for the above-mentioned compound, comprising the following steps: 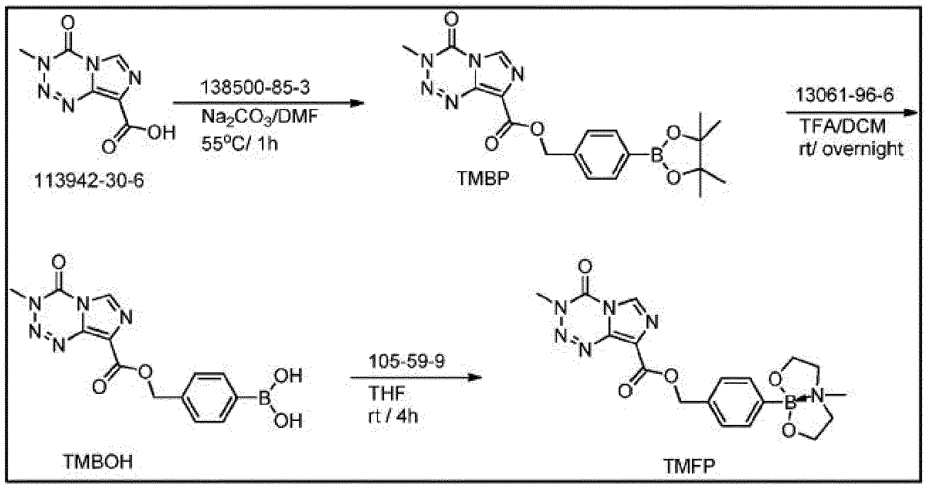
(1) adding 3,4-dihydro-3-methyl-4-oxoimidazo[5,1-d]-1,2,3,5-tetrazine-8-carboxylic acid, 4-(4,4,5,5-tetramethyl-1,3,2-dioxaborolan-2-yl)benzyl bromide and Na₂CO₃ to a flask, and then adding dimethylformamide for dissolution; heating the mixture at 55°C for 60 minutes, after heating stopped, cooling to room temperature; adding water and saturated brine for dilution; then, extracting the mixture with ethyl acetate, rotary evaporating to dryness, and passing through a column with dichloromethane/methanol system to obtain a white solid 3,4-dihydro-3-methyl-4-oxoimidazo[5,1-D]-1,2,3,5-tetrazine-8-carboxylic acid [4-(4,4,5,5-tetramethyl-1,3,2-diazaboran-2-yl)phenyl ester;
(2) adding 3,4-dihydro-3-methyl-4-oxoimidazo[5,1-D]-1,2,3,5-tetrazine-8-carboxylic acid [4-(4,4,5,5-tetramethyl-1,3,2-diazaboran-2-yl)phenyl ester to a flask, and adding a trifluoroacetic acid solution in dichloromethane for dissolution; then, adding methylboronic acid; stirring the mixture at room temperature overnight, rotary evaporating to dryness, and passing through a column with dichloromethane/methanol system to obtain a white solid 3,4-dihydro-3-methyl-4-oxoimidazo[5,1-D]-1,2,3,5-tetrazine-8-methoxy-4-phenylboronic acid;
(3) adding 3,4-dihydro-3-methyl-4-oxoimidazo[5,1-D]-1,2,3,5-tetrazine-8-methoxy-4-phenylboronic acid to a flask, and adding THF for dissolution; then, dropwise adding methyldiethanolamine at room temperature; stirring the mixture at room temperature for 4 hours, rotary evaporating to dryness at low temperature, dissolving with deuterated chloroform, rotary evaporating to dryness again, pumping with oil pump for 2 hours, and quantitatively obtaining a product 3,4-dihydro-3-methyl-4-oxoimidazo[5,1-D]-1,2,3,5-tetrazine-8-methoxy-4-phenylboronic acid ethyl ester, replacing with nitrogen and freezing for storage;
(4) treating with DMF solution containing 20% piperidine for 10 minutes; rinsing with DMF, CH₂Cl₂ and DMF successively; then, adding DMF for dissolution; adding the above mixture to the resin mixture; after reacting for 1 hour, washing with DMF, CH₂Cl₂ and DMF successively, to obtain a target product.

The boron carrying agent and the pharmaceutically acceptable salt thereof according to the present invention can be used in the preparation of a medicament for treating tumors.

The tumor according to the present invention is a malignant tumor or metastatic tumor, preferably cerebral glioma, recurrent head and neck tumor, malignant melanoma, breast cancer or metastatic liver cancer, etc. Malignant tumors are what people often call cancer. When cells in the body mutate, they will continue to divide without being controlled by the body, and eventually form cancer. Malignant tumor cells can invade and destroy adjacent tissues and organs, and the cells can break out of the tumor and enter the blood or lymphatic system. This is how malignant tumors metastasize from the primary site to other organs to form new tumors, which is also called metastatic malignancy.

The tumor according to the present invention is preferably a cerebral tumor or melanoma. Cerebral tumors refer to tumors that grow within the skull, including primary cerebral tumors and secondary cerebral tumors. Melanoma is a highly malignant tumor that produces melanin, which mostly occurs in the skin or mucosae close to the skin, and is also found in the pia mater and choroid.

The cerebral tumor according to the present invention is preferably a cerebral glioma. Generally speaking, tumors originating from neuroepithelium are called cerebral gliomas, accounting for 35-55% of craniocerebral tumors, and are common intracranial malignant tumors.

The boron carrying agent or the pharmaceutically acceptable salt thereof according to the present invention can be used to prepare a pharmaceutical preparation, which comprises:
(1) the boron carrying agent or the pharmaceutically acceptable salt thereof according to the present invention; and
(2) one or more pharmaceutically acceptable carriers or excipients.

Compared with the prior art, the boron carrying agent compound according to the present invention has the advantages of stable quality, has excellent activity compared with the same type of medicines, is suitable for being developed as a novel boron carrying agent, and is suitable for preparing various forms of pharmaceutical formulations. Therefore, the boron carrying agent of the present invention has a good application prospect in preparation of a medicament for treating tumors.

### Brief Description of Drawings

Various other advantages and benefits will become apparent to those skilled in the art upon reading the following detailed description of the preferred embodiments. The drawings are only for the purpose of illustrating the preferred embodiments and should not to be construed as limiting the present invention.
FIG. 1: Detection of the B content in the cells by CP-AES for 12 hours with the boron carrying agent according to the present invention.
FIG. 2: Boron uptake in different tumor cells for 12 hours with BPA.
FIG. 3: Comparison of cellular uptake between the boron carrying agent according to the present invention and BPA for 12 hours.
FIG. 4: Comparison of the uptake between BPA and the boron carrying agents according to the present invention in U87MG, U251, A549 and A375 cell lines.

### Detailed Description of Embodiments

The present invention will be described in further detail below in combination with specific embodiments. The purpose of the embodiments is only to illustrate and describe the current best embodiments of the present invention. The scope of the present invention is not limited in any way by the embodiments described herein.

### Example 1

### Preparation of 3,4-dihydro-3-methyl-4-oxoimidazo[5,1-D]-1,2,3,5-tetrazine-8-carboxylic acid [4-(4,4,5,5-tetramethyl-1,3,2-diaza-borolan-2-yl)phenyl ester

3,4-dihydro-3-methyl-4-oxoimidazo[5,1-d]-1,2,3,5-tetrazine-8-carboxylic acid (200 mg,1.02 mmol), 4-(4,4,5,5-tetramethyl-1,3,2-dioxaborolan-2-yl)benzyl bromide (600 mg, 2.02 mmol) and Na₂CO₃ (170 mg, 1.60 mmol) were added to the flask, and then dimethylformamide (5 mL) was added to dissolve. The mixture was heated at 55°C for 60 minutes, after heating stopped, cooled to room temperature. Water (30 mL) and saturated brine (30 mL) were added to dilute. Then, the mixture was extracted with ethyl acetate (50 mL), rotary evaporated to dryness, and passed through the column with dichloromethane/methanol system to obtain 40 mg of white solid 3,4-dihydro-3-methyl-4-oxoimidazo[5,1-D]-1,2,3,5-tetrazine-8-carboxylic acid [4-(4,4,5,5-tetramethyl-1,3,2-diazaboran-2-yl)phenyl ester.

### Example 2

### Preparation of 3,4-dihydro-3-methyl-4-oxoimidazo[5,1-D]-1,2,3,5-tetrazine-8-methoxy-4-phenylboronic acid

3,4-dihydro-3-methyl-4-oxoimidazo[5,1-D]-1,2,3,5-tetrazine-8-carboxylic acid [4-(4,4,5,5-tetramethyl-1,3,2-diazaboran-2-yl)phenyl ester (200 mg, 0.486 mmol) was added to the flask, and trifluoroacetic acid solution in dichloromethane (5 mL, 5%) was added to dissolve. Then, methylboronic acid (300 mg, 5 mmol) was added. The mixture was stirred at room temperature overnight, rotary evaporated to dryness, and passed through the column with dichloromethane/methanol system to obtain 130 mg of white solid 3,4-dihydro-3-methyl-4-oxoimidazo[5,1-D]-1,2,3,5-tetrazine-8-methoxy-4-phenylboronic acid.

### Example 3

### Preparation of 3,4-dihydro-3-methyl-4-oxoimidazo[5,1-D]-1,2,3,5-tetrazine-8-methoxy-4-phenylboronic acid ethyl ester

3,4-dihydro-3-methyl-4-oxoimidazo[5,1-D]-1,2,3,5-tetrazine-8-methoxy-4-phenylboronic acid (100 mg, 0.304 mmol) was added to the flask, and THF (3 mL) was added to dissolve. Then, methyldiethanolamine (36.2 mg, 0.304 mmol) was dropwise added at room temperature. The mixture was stirred at room temperature for 4 hours, rotary evaporated to dryness at low temperature, dissolved with deuterated chloroform, rotary evaporated to dryness again, pumped with oil pump for 2 hours, and quantitatively obtain the product 3,4-dihydro-3-methyl-4-oxoimidazo[5,1-D]-1,2,3,5-tetrazine-8-methoxy-4-phenylboronic acid ethyl ester (125.1 mg), replacing with nitrogen and freezing for storage.

DMF solution containing 20% piperidine was used for treatment for 10 minutes. DMF, CH₂Cl₂ and DMF were successively used for rinsing. Then, 700 µL DMF was added to dissolve. The above mixture was added to the resin mixture. After reacting for 1 hour, DMF, CH₂Cl₂ (2x) and DMF were successively used for washing, to obtain the target product of the present invention. Measured: ¹NHR (400MHz, Chlorogorm-d) δ8.85(s, 1H), 7.54-7.48 (m, 2H), 7.34(d, *J*=7.8Hz, 2H), 5.39(s.2H), 3.97-3.84(m, 7H), 3.24(ddd, J=11.6, 5.4, 4.1Hz, 2H), 2.95 (ddd, *J*=11.6, 8.3, 6.5Hz, 2H) 2.20 (s, 3H).

### Example 4

In vitro study on the compound according to the present invention

3,4-dihydro-3-methyl-4-oxoimidazo[5,1-D]-1,2,3,5-tetrazine-8-methoxy-4-phenylboronic acid ethyl ester was formulated into aqueous solutions of 0.1 mg/g, 0.2 mg/g, 0.4 mg/g, 0.6 mg/g and 0.8 mg/g, added to U87MG, U251, A549 and A375 cell lines cultured in 25T petri dishes, respectively, and incubated in a thermostatic incubator for 12 hours. Subsequently, the cells and culture medium were separated, and the number of cells was determined. After the cells were digested with concentrated nitric acid, the B content in the cells was determined by ICP-AES.

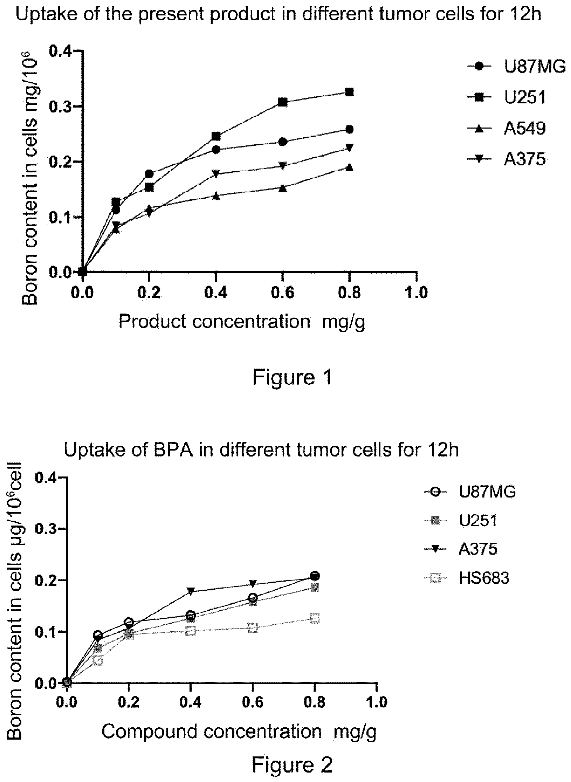

The detection limit was 249.772 nm. After determination, the B content contained in every 10⁶ cells can be calculated and plotted, as shown in Figure 1. As can be seen, within 12 hours, the boron concentration in the cells is positively correlated with the concentration of the added product, with increasing trend gradually slowing down. Within 12 hours, best uptake of the products was observed in the glioma cell line U251.

Meanwhile, the BPA was formulated into aqueous solutions of 0.1 mg/g, 0.2 mg/g, 0.4 mg/g, 0.6 mg/g, and 0.8 mg/g, added into U87MG, U251, A375 and HS683 cell lines cultured in 25T petri dishes, respectively, and cultured in a thermostatic incubator for 12 hours. Subsequently, the cells and culture medium were separated, and the number of cells was determined. After the cells were digested with concentrated nitric acid, the B content in the cells was determined by ICP-AES and plotted as shown in Figure 2.

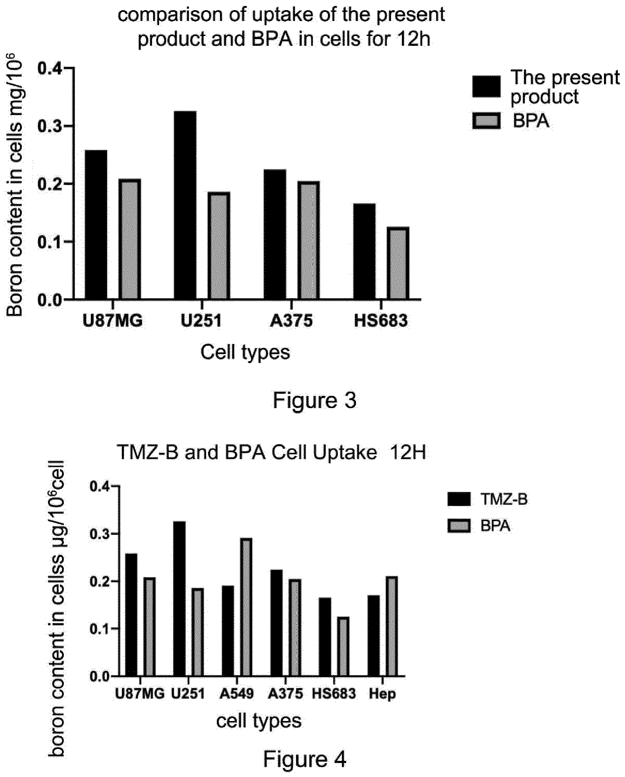

Comparison between Figure 2 and Figure 3 shows that among the uptake in cerebral glioma HS683, U251 and U87MG cells, the present product was significantly higher than BPA, as shown in Figure 3.

### Example 5

In vivo study on the compound according to the present invention

Healthy BALB/c mice with an average weight of approximately 21 g were selected and inoculated with HS683 cerebral glioma to establish a mouse cerebral glioma model. After 21 days, the mouse cerebral glioma model was generated, and 500 mg/kg of BPA, temozolomide or 3,4-dihydro-3-methyl-4-oxoimidazo[5,1-D]-1,2,3,5-tetrazine-8-methoxy-4-phenylboronic acid ethyl ester solution was injected via the tail vein of the mice. After administration, the major tissues and organs (heart, liver, spleen, lungs, kidneys and tumor sites) of the mice were removed at selected time periods (1 hour, 2 hours, and 4 hours). After washing and weighing, the tissues and organs were digested with concentrated nitric acid. The digested tissue sample was diluted and the concentration in the sample was determined by ICP-MS.

As can be seen, two hours after administration, the uptake of the present product is higher than that of BPA in the mouse cerebral glioma site (see Figure 4), indicating that the present product can improve the targeting effect of the boron carrying agent and increase the effective neutron absorption during BNCT irradiation.

## Claims

1. A boron carrying agent or a pharmaceutically acceptable salt thereof, comprising the structure of:

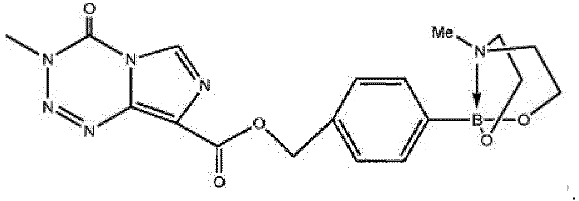

2. Use of the boron carrying agent according to claim 1 in the preparation of a medicine for treating a tumor.

3. The use according to claim 2, wherein the tumor is a malignant tumor or metastatic tumor.

4. The use according to claim 3, wherein the tumor is cerebral tumor, recurrent head and neck tumor, malignant melanoma, breast cancer or metastatic liver cancer.

5. The use according to claim 4, wherein the tumor is preferably cerebral glioma or malignant melanoma.

6. A pharmaceutical formulation comprising:
(1) the boron carrying agent or the pharmaceutically acceptable salt thereof according to claim 1; and
(2) one or more pharmaceutically acceptable carriers or excipients.

7. Use of the pharmaceutical formulation according to claim 6 in the preparation of a medicine for treating a tumor.

8. The use according to claim 7, wherein the tumor is a malignant tumor or metastatic tumor.

9. The use according to claim 8, wherein the tumor is cerebral glioma, recurrent head and neck tumor, malignant melanoma, breast cancer or metastatic liver cancer.

10. A method for preparing the boron carrying agent according to claim 1, comprising the following steps:
(1) adding 3,4-dihydro-3-methyl-4-oxoimidazo[5,1-d]-1,2,3,5-tetrazine-8-carboxylic acid, 4-(4,4,5,5-tetramethyl-1,3,2-dioxaborolan-2-yl)benzyl bromide and Na₂CO₃ to a flask, and then adding dimethylformamide for dissolution; heating the mixture at 55°C for 60 minutes, after heating stopped, cooling to room temperature; adding water and saturated brine for dilution; then, extracting the mixture with ethyl acetate, rotary evaporating to dryness, and passing through a column with dichloromethane/methanol system to obtain a white solid 3,4-dihydro-3-methyl-4-oxoimidazo[5,1-D]-1,2,3,5-tetrazine-8-carboxylic acid [4-(4,4,5,5-tetramethyl-1,3,2-diazaboran-2-yl)phenyl ester;
(2) adding 3,4-dihydro-3-methyl-4-oxoimidazo[5,1-D]-1,2,3,5-tetrazine-8-carboxylic acid [4-(4,4,5,5-tetramethyl-1,3,2-diazaboran-2-yl)phenyl ester to a flask, and adding a trifluoroacetic acid solution in dichloromethane for dissolution; then, adding methylboronic acid; stirring the mixture at room temperature overnight, rotary evaporating to dryness, and passing through a column with dichloromethane/methanol system to obtain a white solid 3,4-dihydro-3-methyl-4-oxoimidazo[5,1-D]-1,2,3,5-tetrazine-8-methoxy-4-phenylboronic acid;
(3) adding 3,4-dihydro-3-methyl-4-oxoimidazo[5,1-D]-1,2,3,5-tetrazine-8-methoxy-4-phenylboronic acid to a flask, and adding THF for dissolution; then, dropwise adding methyldiethanolamine at room temperature; stirring the mixture at room temperature for 4 hours, rotary evaporating to dryness at low temperature, dissolving with deuterated chloroform, rotary evaporating to dryness again, pumping with oil pump for 2 hours, and quantitatively obtaining a product 3,4-dihydro-3-methyl-4-oxoimidazo[5,1-D]-1,2,3,5-tetrazine-8-methoxy-4-phenylboronic acid ethyl ester, replacing with nitrogen and freezing for storage;
(4) treating with DMF solution containing 20% piperidine for 10 minutes; rinsing with DMF, CH₂Cl₂ and DMF successively; then, adding DMF for dissolution; adding the above mixture to the resin mixture; after reacting for 1 hour, washing with DMF, CH₂Cl₂ and DMF successively, to obtain a target product.

## Patentansprüche

1. Ein Bor-Träger oder ein pharmazeutisch verträgliches Salz davon, umfassend die folgende Struktur

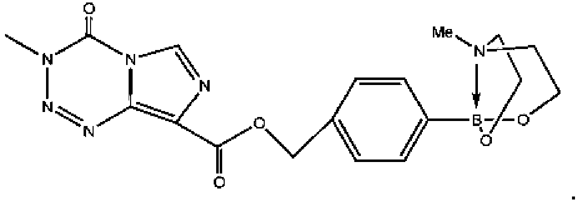

2. Verwendung des Bor-Trägers nach Anspruch 1 zur Herstellung eines Arzneimittels zur Behandlung eines Tumors.

3. Verwendung nach Anspruch 2, wobei es sich bei dem Tumor um einen malignen oder metastasierten Tumor handelt.

4. Verwendung nach Anspruch 3, wobei es sich bei dem Tumor um einen Hirntumor, einen rezidivierenden Kopf-Hals-Tumor, ein malignes Melanom, Brustkrebs oder Lebermetastasen handelt.

5. Verwendung nach Anspruch 4, wobei es sich bei dem Tumor vorzugsweise um ein zerebrales Gliom oder ein malignes Melanom handelt.

6. Eine pharmazeutische Formulierung, umfassend:
(1) den Bor-Träger oder das pharmazeutisch verträgliche Salz davon nach Anspruch 1; und
(2) einen oder mehrere pharmazeutisch verträgliche Träger oder Hilfsstoffe.

7. Verwendung der pharmazeutischen Formulierung nach Anspruch 6 zur Herstellung eines Arzneimittels zur Behandlung eines Tumors.

8. Verwendung nach Anspruch 7, wobei es sich bei dem Tumor um einen malignen oder metastasierten Tumor handelt.

9. Verwendung nach Anspruch 8, wobei es sich bei dem Tumor um ein zerebrales Gliom, einen rezidivierenden Kopf-Hals-Tumor, ein malignes Melanom, Brustkrebs oder Lebermetastasen handelt.

10. Verfahren zur Herstellung des Bor-Trägermittels nach Anspruch 1, umfassend die folgenden Schritte:
(1) Vorlegen von 3,4-Dihydro-3-methyl-4-oxoimidazo[5,1-d]-1,2,3,5-tetrazin-8-carbonsäure, 4-(4,4,5,5-Tetramethyl-1,3,2-dioxaborolan-2-yl)benzylbromid und Na₂CO₃ in einen Kolben und anschließendes Zugeben von Dimethylformamid zum Lösen; Erhitzen der Mischung für 60 Minuten auf 55 °C, nach Beendigung der Heizung Abkühlen auf Raumtemperatur; Zugabe von Wasser und gesättigter Kochsalzlösung zum Verdünnen; Extrahieren des Gemisches mit Ethylacetat, Eindampfen zur Trockne im Rotationsverdampfer und Säulenchromatographie mit einem Dichlormethan/Methanol-System, um einen weißen Feststoff, 3,4-Dihydro-3-methyl-4-oxoimidazo[5, 1-D]-1 ,2,3,5-tetrazin-8-carbonsäure-[4-(4,4,5,5-tetramethyl-1 ,3,2-diazaboran-2-yl)phenylester], zu erhalten;
(2) Vorlegen von 3,4-Dihydro-3-methyl-4-oxoimidazo[5,1-D]-1,2,3,5-tetrazin-8-carbonsäure[4-(4,4,5,5-tetramethyl-1,3,2-diazaboran-2-yl)phenylester] in einen Kolben und Zugabe einer Trifluoressigsäurelösung in Dichlormethan zum Auflösen; anschließend Zugabe von Methylboronsäure; Rühren der Mischung über Nacht bei Raumtemperatur, Eindampfen im Rotationsverdampfer zur Trockne und Elution über eine Säule mit einem Dichlormethan/Methanol-System, um einen weißen Feststoff, 3,4-Dihydro-3-methyl-4-oxoimidazo[5,1-D]-1,2,3,5-tetrazin-8-methoxy-4-phenylboronsäure, zu erhalten;
(3) Zugabe von 3,4-Dihydro-3-methyl-4-oxoimidazo[5,1-D]-1,2,3,5-tetrazin-8-methoxy-4-phenylboronsäure in einen Kolben, und Zugabe von THF zum Auflösen; anschließend tropfenweise Zugabe von Methyldiethanolamin bei Raumtemperatur; Rühren der Mischung 4 Stunden lang bei Raumtemperatur, Eindampfen zur Trockne bei niedriger Temperatur, Lösen in deuteriertem Chloroform, erneutes Eindampfen zur Trockne, 2 Stunden lang Pumpen mit einer Ölpumpe, und quantitative Gewinnung des Produkts 3,4-Dihydro-3-methyl-4-oxoimidazo[5, 1-D]-1 ,2,3,5-tetrazin-8-methoxy-4-phenylboronsäureethylester, Ersetzen des Gases durch Stickstoff und Einfrieren zur Lagerung;
(4) Behandeln mit einer DMF-Lösung, die 20 % Piperidin enthält, für 10 Minuten; sukzessives Waschen mit DMF, CH₂Cl₂ und DMF; anschließendes Zugeben von DMF zum Auflösen; Zugeben der obigen Mischung zur Harzmischung; nach einstündiger Reaktionszeit, sukzessives Waschen mit DMF, CH₂Cl₂ und DMF, um das Zielprodukt zu erhalten.

## Revendications

1. Agent porteur de bore ou un sel pharmaceutiquement acceptable de celui-ci, comprenant la structure suivante :

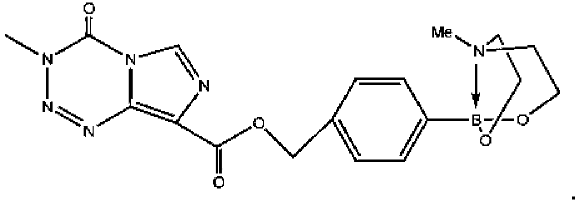

2. Utilisation de l'agent porteur de bore selon la revendication 1 dans la préparation d'un médicament pour le traitement d'une tumeur.

3. Utilisation selon la revendication 2, dans laquelle la tumeur est une tumeur maligne ou une tumeur métastatique.

4. Utilisation selon la revendication 3, dans laquelle la tumeur est une tumeur cérébrale, une tumeur récidivante de la tête et du cou, un mélanome malin, un cancer du sein ou un cancer du foie métastatique.

5. Utilisation selon la revendication 4, dans laquelle la tumeur est de préférence un gliome cérébral ou un mélanome malin.

6. Formulation pharmaceutique comprenant :
(1) l'agent porteur de bore ou un sel pharmaceutiquement acceptable de celui-ci selon la revendication 1 ; et
(2) un ou plusieurs excipients ou supports pharmaceutiquement acceptables.

7. Utilisation de la formulation pharmaceutique selon la revendication 6 dans la préparation d'un médicament pour le traitement d'une tumeur.

8. Utilisation selon la revendication 7, dans laquelle la tumeur est une tumeur maligne ou une tumeur métastatique.

9. Utilisation selon la revendication 8, dans laquelle la tumeur est un gliome cérébral, une tumeur récidivante de la tête et du cou, un mélanome malin, un cancer du sein ou un cancer du foie métastatique.

10. Procédé de préparation de l'agent porteur de bore selon la revendication 1, comprenant les étapes suivantes :
(1) introduction dans un ballon de l'acide 3,4-dihydro-3-méthyl-4-oxoimidazo[5,1-d]-1,2,3,5-tétrazine-8-carboxylique, du bromure de 4-(4,4,5,5-tétraméthyl-1,3,2-dioxaborolan-2-yl)benzyle et du Na₂CO₃, puis ajout de diméthylformamide pour dissolution ; chauffage du mélange à 55 °C pendant 60 minutes, puis refroidissement à température ambiante après arrêt du chauffage ; dilution par ajout d'eau et de saumure saturée ; extraction du mélange avec de l'acétate d'éthyle, évaporation rotative à sec et passage à travers une colonne avec un système dichlorométhane/méthanol pour obtenir un solide blanc, l'acide 3,4-dihydro-3-méthyl-4-oxoimidazo[5,1-D]-1,2,3,5-tétrazine-8-carboxylique [4-(4,4,5,5-tétraméthyl-1,3,2-diazaboran-2-yl)phényl ester ;
(2) introduction de l'acide 3,4-dihydro-3-méthyl-4-oxoimidazo[5,1-D]-1,2,3,5-tétrazine-8-carboxylique [4-(4,4,5,5-tétraméthyl-1,3,2-diazaboran-2-yl)phényl ester] dans un ballon, et ajout d'une solution d'acide trifluoroacétique dans le dichlorométhane pour dissolution ; puis, ajout d'acide méthylboronique ; agitation du mélange à température ambiante pendant une nuit, évaporation rotative à sec, et passage sur une colonne avec un système dichlorométhane/méthanol pour obtenir un solide blanc d'acide 3,4-dihydro-3-méthyl-4-oxoimidazo[5,1-D]-1,2,3,5-tétrazine-8-méthoxy-4-phénylboronique ;
(3) introduction de l'acide 3,4-dihydro-3-méthyl-4-oxoimidazo[5,1-D]-1,2,3,5-tétrazine-8-méthoxy-4-phénylboronique dans un ballon, et ajout de THF pour dissolution ; puis, ajout goutte à goutte de méthyldiéthanolamine à température ambiante ; agitation du mélange à température ambiante pendant 4 heures, évaporation à sec sous vide à basse température, dissolution dans du chloroforme deutéré, nouvelle évaporation à sec sous vide, mise sous vide à l'aide d'une pompe à huile pendant 2 heures, et obtention quantitative de l'ester éthylique de l'acide 3,4-dihydro-3-méthyl-4-oxoimidazo[5,1-D]-1,2,3,5-tétrazine-8-méthoxy-4-phénylboronique, remplacement du gaz par de l'azote et congélation pour stockage ;
(4) traitement avec une solution de DMF contenant 20 % de pipéridine pendant 10 minutes ; rinçages successifs avec du DMF, du CH₂Cl₂ et du DMF ; ajout de DMF pour dissolution ; ajout du mélange obtenu au mélange de résine ; après une heure de réaction, lavages successifs avec du DMF, du CH₂Cl₂ et du DMF pour obtenir le produit cible.
